# EUROPEAN PATENT APPLICATION

(11) **EP 4 847 638 A2**
(43) Date of publication of application: **02.12.2026**
(21) Application number: 26181747.2
(22) Date of filing: 28.05.2026
(51) Int. Cl.: C21D 1/18, A61B 17/00, A61C 1/00, A61C 17/00, B23B 37/00, B24B 1/00, C21D 1/26, C21D 1/74, C21D 9/22

(54) **HEAT TREATMENT METHOD AND SYSTEM FOR REPLACEABLE TOOL TIPS OF ULTRASONIC IMPACT GRINDING**

(30) Priority: 28.05.2025 US 202519220635
(71) Applicant: RTX Corporation, Farmington, CT 06032 (US)
(72) Inventor: WANG, Zhigang, South Windsor, CT 06074 (US); KUCZEK, Andrzej E., Bristol, CT 06010 (US); FERNANDEZ, Robin H., Cornwall, CT 06759 (US); BARRON, Alan C., Jupiter, FL 33458 (US); ABDI, Ahmed Abdillahi, San Diego, CA 92115 (US)
(74) Representative: Dehns

(57) **Abstract**

An ultrasonic vibration tool is used to machine a workpiece, such as a ceramic matrix composite (CMC) preform. The ultrasonic vibration tool includes a transducer, a horn, and an ultrasonic tool having a tool tip. The tool tip is made of steel, such as D2 steel, while the horn is made of a titanium alloy. The tool tip is subject to a hardening process that preheats the tool tip in a furnace then heats the tool tip at a hardening temperature. The tool tip is then placed in a vacuum furnace to cool to a cooled temperature. The tool tip is subject to a tempering process for a tempering period to target a balance of hardness and toughness.

## Description

### FIELD OF THE INVENTION

The subject matter disclosed herein relates to the use of replaceable tool tips in ultrasonic impact grinding processes. In particular, the subject matter relates to treatment of the replaceable tool tips to improve wear resistance of the tips.

### BACKGROUND OF THE INVENTION

Ceramic matrix composites (CMCs) may be formed of silicon carbide (SiC) fibers in a SiC matrix. The hardness of SiC/SiC CMCs is second only to that of diamond tooling. Additionally, the SiC fiber reinforced phase creates anisotropy and heterogeneity material properties. Therefore, it has been a challenge to develop an efficient and cost-effective way to perform high quality machining on such CMCs.

The ultrasonic impact grinding (UIG) process may offer three-dimensional machining capability to machine a variety of materials, such as CMCs. A factor in the UIG process is tool wear of tools used to implement the grinding. Tool wear decreases the material removal rate, which, in turn, reduces machining performance and accuracy. Thus, it may be appreciated that a method may be needed to balance the hardness and the toughness of a tool tip used in UIG processing to improve wear resistance.

### SUMMARY OF THE INVENTION

The present disclosure is directed, in a first aspect, to a method for treating a tool tip of an ultrasonic vibration tool. The method includes preheating the tool tip to an initial preheat temperature within a furnace. The method also includes heating the tool tip to a final preheat temperature from the initial preheat temperature within the furnace. The method also includes increasing heat within the furnace to a hardening temperature for the tool tip for a hardening period of time. The method also includes placing the tool tip into a vacuum furnace until the tool tip is cooled to a cooled temperature. The method also includes heating the tool tip to a tempering temperature in the furnace for a tempering period. The method also includes allowing the tool tip to cool in the furnace to a room temperature to achieve a hardness of a Rockwell Hardness Scale C (HRC) value between 58 and 62.

In some embodiments, the tool tip is comprised of tool steel. In further embodiments, the tool tip is comprised of one of H13, P20, and D2 steel. In some embodiments, the initial preheat temperature is between 640 degrees and 650 degrees Celsius. In some embodiments, preheating the tool tip includes increasing a temperature within the furnace to the initial preheat at a specified increment per hour.

In some embodiments, the final preheat temperature is between 780 degrees and 790 degrees Celsius. In some embodiments, the hardening temperature is between 980 degrees and 990 degrees Celsius. In some embodiments, the cooled temperature is between 60 degrees and 70 degrees Celsius.

In some embodiments, the tempering temperature is between 270 degrees and 280 degrees Celsius. In some embodiments, the tempering period corresponds to a thickness of the tool tip. In some embodiments, the vacuum furnace includes a sealed chamber to hold the tool tip.

In yet another embodiment, the present disclosure is directed to an ultrasonic vibration tool. The ultrasonic vibration tool includes a transducer configured to convert electrical energy into mechanical vibrations. The ultrasonic vibration tool also includes a horn coupled to the transducer to amplify the mechanical vibrations. The ultrasonic vibration tool also includes an ultrasonic tool coupled to the horn to move along a longitudinal axis of the ultrasonic vibration tool according to the mechanical vibrations. The ultrasonic tool includes a tool tip comprised of heat treated steel.

In some embodiments, the tool tip is replaceable. In some embodiments, the steel comprises tool steel. In some embodiments, the horn is comprised of a titanium alloy.

In yet another embodiment, the present disclosure is directed to an ultrasonic machining system. The ultrasonic machining system includes an ultrasonic vibration tool. The ultrasonic vibration tool includes a transducer configured to convert electrical energy into mechanical vibrations. The ultrasonic vibration tool also includes a horn coupled to the transducer to amplify the mechanical vibrations. The ultrasonic vibration tool also includes an ultrasonic tool coupled to the horn to move along a longitudinal axis of the ultrasonic vibration tool according to the mechanical vibrations. The ultrasonic tool includes a tool tip comprised of heat-treated steel. The ultrasonic machining system also includes a slurry component having a slurry nozzle to deliver a slurry having abrasive particles in an area of the tool tip. The tool tip engages the abrasive particles to machine a workpiece. The ultrasonic machining system also includes an ultrasonic power supply to provide the electrical energy to the ultrasonic vibration tool.

In some embodiments, the tool tip is replaceable. In some embodiments, the tool tip comprises tool steel. In some embodiments, the horn comprises a titanium alloy. In additional embodiments, the ultrasonic machining system also includes a control unit to control the ultrasonic vibration tool.

### BRIEF DESCRIPTION OF FIGURES

The features of the disclosure believed to be novel and the elements characteristic of the invention are set forth with particularity in the appended claims. The figures are for illustration purposes only and are not drawn to scale. The disclosure itself, however, both as to organization and method of operation, can best be understood by reference to the description of the preferred embodiment(s) which follows, taken in conjunction with the accompanying drawings in which:
Figure 1 illustrates an ultrasonic vibration tool according to the disclosed embodiments.
Figure 2 illustrates a machining process implemented by the ultrasonic vibration tool according to the disclosed embodiments.
Figure 3 illustrates a weaving pattern for a CMC according to the disclosed embodiments.
Figure 4 illustrates a perspective view of the ultrasonic vibration tool having a replaceable tool tip according to the disclosed embodiment.
Figure 5 illustrates a flowchart for hardening a replaceable tool tip according to the disclosed embodiments.
Figure 6 illustrates a block diagram of a system for hardening the replaceable tool tip according to the disclosed embodiments.
Figure 7 illustrates a flowchart for tempering the replaceable tool tip according to the disclosed embodiments.
Figure 8 illustrates a graph of the tool wear comparison between annealed/as received tool tips versus heat treated tool tips according to the disclosed embodiments.
Figure 9 illustrates a block diagram of an ultrasonic machining system using the ultrasonic vibration tool according to the disclosed embodiments.
Figure 10 illustrates a block diagram of a one embodiment of a control unit within the ultrasonic tooling system according to the disclosed embodiments.

### DETAILED DESCRIPTION OF THE INVENTION

The embodiments of the present disclosure can comprise, consist of, and consist essentially of the features and/or steps described herein, as well as any of the additional or optional ingredients, components, steps, or limitations described herein or would otherwise be appreciated by one of skill in the art.

The following description omits or only briefly describes conventional features of the disclosed technology that are apparent to those skilled in the art. Reference to a particular embodiment does not limit the scope of the claims attached hereto. Additionally, any examples set forth in this specification are intended to be non-limiting and merely set forth some of the many possible embodiments for the appended claims. Further, particular features described herein can be used in combination with other described features in each of the various possible combinations and permutations. A person of ordinary skill in the art would know how to use the instant invention, in combination with routine experiments, to achieve other outcomes not specifically disclosed in the examples or the embodiments.

Unless otherwise specifically defined herein, all terms are to be given their broadest possible interpretation including meanings implied from the specification as well as meanings understood by those skilled in the art and/or as defined in dictionaries, treatises, etc. Unless defined otherwise, all technical and scientific terms used herein have the same meaning as commonly understood by one of ordinary skill in the art in the field of the disclosed technology. It must also be noted that, as used in the specification and the appended claims, the singular forms "a," "an," and "the" include plural referents unless otherwise specified, and that the terms "includes" and/or "including," when used in this specification, specify the presence of stated features, elements, and/or components, but do not preclude the presence or addition of one or more other features, steps, operations, elements, components, and/or groups thereof. Additionally, methods, equipment, and materials similar or equivalent to those described herein can also be used in the practice or testing of the disclosed technology.

The devices of the present disclosure may be understood more readily by reference to the following detailed description of the embodiments taken in connection with the accompanying drawing figures, which form a part of this disclosure. It is to be understood that this application is not limited to the specific devices, methods, conditions or parameters described and/or shown herein, and that the terminology used herein is for the purpose of describing particular embodiments by way of example only and is not intended to be limiting. All spatial references, such as, for example, proximal, distal, horizontal, vertical, top, upper, lower, bottom, left and right, are for illustrative purposes only and can be varied within the scope of the disclosure. For example, the references "upper" and "lower" are relative and used only in the context to the other, and are not necessarily "superior" and "inferior."

It will further be understood that, although the terms "first," "second," "third," and the like may be used herein to describe various elements, these elements should not be limited by these terms. These terms are only used to distinguish one element from another element. Thus, "a first element" discussed below could be termed "a second element" or "a third element," and "a second element" and "a third element" may be termed likewise without departing from the teachings herein.

The present disclosure is directed to methods and system to increase tool wear resistance that improves UIG machining performance. The disclosed embodiments implement heat treatment to balance the tool hardness and toughness to improve tool wear resistance. The tool of the disclosed embodiments includes replaceable tool tips. The replaceable tool tips are made of D2 steel. The tool tips may use D2 steel instead of titanium. D2 steel is made at an annealed status, which has the hardness in Rockwell C (HRC) 29. The lower hardness may contribute to tool wear. To maximize hardness and wear resistance, the disclosed embodiments harden the tool tip and reduce the internal stress via tempering. With the disclosed heat treatment process, it is feasible to balance tool hardness and toughness to improve tool wear resistance.

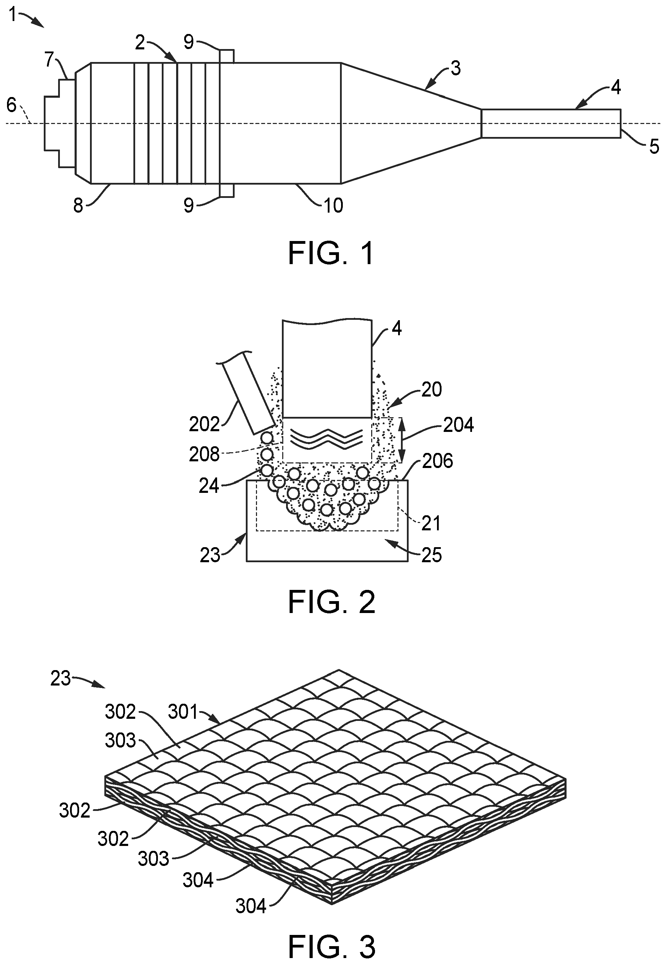

Figure 1 depicts an ultrasonic vibration tool 1 according to the disclosed embodiments. Ultrasonic vibration tool 1 includes a transducer 2, an energy-forming horn 3, an ultrasonic tool 4, and a tool tip 5. The electrical energy input to transducer 2 is converted to mechanical vibrations at a high frequency (usually at 20-40 kHz) along longitudinal axis 6 of vibration tool 1. The excited vibration is subsequently transmitted through energy-focusing horn 3 to tool 4 in order to amplify the vibration amplitude which is delivered to tool tip 5. Vibration tool 1 is located directly above the workpiece (not shown in Figure 1) and vibrates along its longitudinal axis 6 at a desired amplitude.

Ultrasonic vibration tool 1 is used to machine a CMC component such as to form holes, slots, and the like. Vibration tool 1 is attached to a work fixture (not shown) using a suitable fastener 7. Vibration tool 1 also includes back portion 8, flange 9 and front portion 10 that enhance delivery of the mechanical vibrations from horn 3 to tool 4. These features may be arranged in various configurations to achieve the functionality disclosed above.

Figure 2 depicts a machining process implemented by ultrasonic vibration tool 1 according to the disclosed embodiments. Ultrasonic tool 4 is vibrated toward and away from a surface 206 of workpiece 23 in a direction 204 that is, for example, perpendicular to the surface of the workpiece. In some embodiments, tool 4 may be vibrated toward and away at other angles related to workpiece 23. Workpiece 23 may be a substrate that is part of the CMC component. An abrasive slurry 20 is constantly fed into machining area 21 by slurry source 202. Slurry 20 contains abrasive particles 24, made of abrasive material, such as diamond, boron carbide, and the like, that is suspended in water, oil, or other solutions. Slurry 20 also flushes away debris from machining area 21.

The vibration of tool 4 causes abrasive particles 24 contained in slurry 20 located between the tool and workpiece 23 to impact surface 206 and subsequent surfaces, thereby the removal of material by microchipping as generally indicated by reference number 25 in Figure 2. Because the actual machining is carried out by abrasive particles 24, tool 4 may be softer than the workpiece 23. Thus, the UIG process can be treated as a micro scale material fracture process using abrasive particles 24 as the cutting edges.

Material removal rate mainly depends on vibration amplitude 208, applied static pressure, abrasive concentration, and size distribution of the abrasive particles. Once the slurry solution is selected to be used, primarily only vibration amplitude 208 and feed rates can be adjusted to achieve the desired material removal. Vibration amplitude 208 refers to the peak-to-peak amplitude at tool tip 5 of tool 4. Tool tip 5 vibrates in direction 204. The amplitude of this vibration, or vibration amplitude 208, varies as tool 1 operates.

Figure 3 depicts a microstructure of a workpiece 23 comprised of CMC material 301 having fiber tows 302 and 303 which are formed of CMC fiber, and are interwoven. Workpiece 23 may be an intermediate product preform being machined to form a component, such as a blade outer air seal, a static vane, a turbine blade, other components for gas turbine engines, and other components for other applications. CMC material 301 also can include a CMC matrix such as shown at 304, as well as pores or voids in the matrix.

The UIG process may be used for drilling holes and for machining of slots and pockets. Hole drilling is a known machining process for product manufacturing. The UIG process provides an increased potentiality in fabricating diverse holes, especially those with small diameters and high aspect ratios on hard and brittle materials, such as CMCs. Tool wear may impact the material removal process. The complex tool wear patterns in UIG processes may be divided into longitudinal wear and lateral wear. Tool wear not only reduces machining performance but also the accuracy of machined features. For example, a worn tool may produce a poor geometrical accuracy. A wrong choice of tool may lead to failure of the machining.

UIG processes wear down ultrasonic tool 4 and workpiece 23. The wear ratio of these components may be defined as the ratio between the loss in length of tool 4 and the depth of the cavity cut in workpiece 23. For example, for a CMC part and D2 steel tool, tool 4 may be shortened by 0.001 inch after a cavity having a depth of 0.050 inch is formed in the CMC. Thus, the wear ratio may be 1 to 50.

The tool material used in the UIG processes impacts the material removal rate. Tool materials may have a high wear resistance as well as good elastic and fatigue strength properties. Tool materials also may have improved values of toughness and hardness based on a specific application. Stress also may be an important consideration with regards to an ultrasonic vibration tool. In ultrasonic systems, the number of vibration cycles rises quickly, such as a million cycles in 50 seconds at 20 kHz. Fatigue is a likely mode of failure. Stress should be kept below the fatigue limit for the material used in the tool. Acoustic properties of materials used in ultrasonic applications also are a consideration. Materials having favorable acoustic properties, however, may not be so favorable as tooling materials.

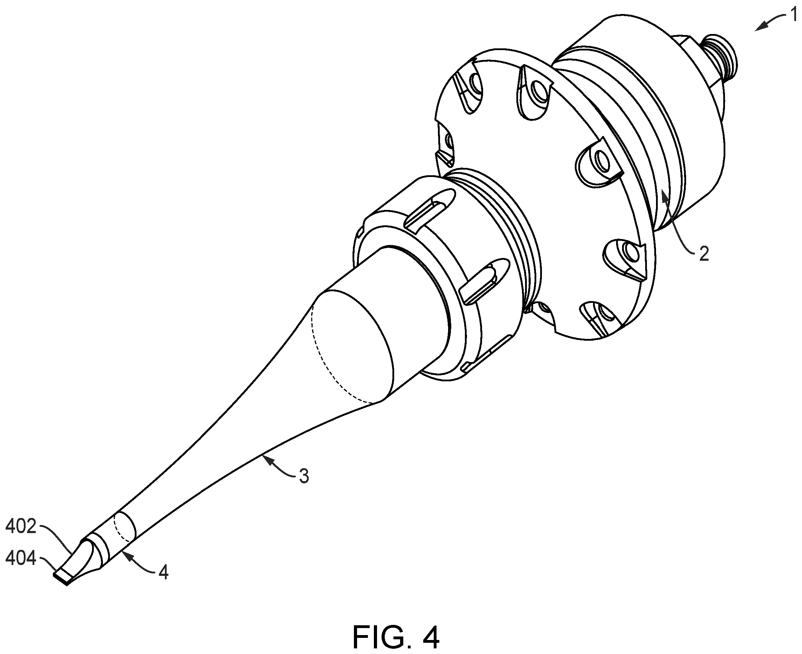

Figure 4 depicts a perspective view of ultrasonic vibration tool 1 having a replaceable tool tip 402 according to the disclosed embodiment. Replaceable tool tip 402 corresponds to tool tip 5, as disclosed above. Replaceable tool tip 402 is attached to ultrasonic tool 4. Ultrasonic tool 4 works in conjunction with transducer 2 and horn 3 to provide the mechanical vibrations for machining workpiece 23. Replaceable tool tip 402 includes tool edge 404 that engages workpiece 23.

The material for horn 3 may be one that provides good acoustic characteristics while having an acceptable level of hardness, or Rockwell Hardness Scale C (HRC). For example, a titanium alloy may be used as the material for horn 3. In some embodiments, Ti6AI4V may be used as the material for horn 3. In some embodiments, the material used for tool tip 402 differs from the material used for horn 3. Thus, tool tip 402 may not be made of Ti6Al4V due to properties associated with using a diamond slurry with a concentration ratio of 5% to 20%.

In some embodiments, the material used for tool tip 402 is tool steel. The types of tool steel may include H13, P20, or D2 steel. In other embodiments, stainless steel and lower carbon steel also may be used. Drilling loads may be more stable with tool tips made of tool steel than titanium alloy. With tool steel tool tips, diamond abrasive particles tend to penetrate the tool bottom surface, or tool edge 404, which creates a strong bond between the diamond abrasives and the steel substrate. This feature makes the steel surface highly abrasive and suitable for cutting or grinding hard materials due to the exceptional hardness of the diamond particles, disclosed as particles 24 above. Other materials may not have such good fusion to the diamond particles as tool steel, such as D2 steel.

Further improvements to tool tip 402 may be realized according to the disclosed embodiments. The disclosed embodiments improve the tooling wear resistance and material removal rate using a heat treatment method to achieve a good combination of fatigue strength and hardness. For example, the disclosed process may increase the hardness of D2 annealed steel from HRC 29 to HRC 60, which provides a desired balance of hardness/wear resistance and toughness.

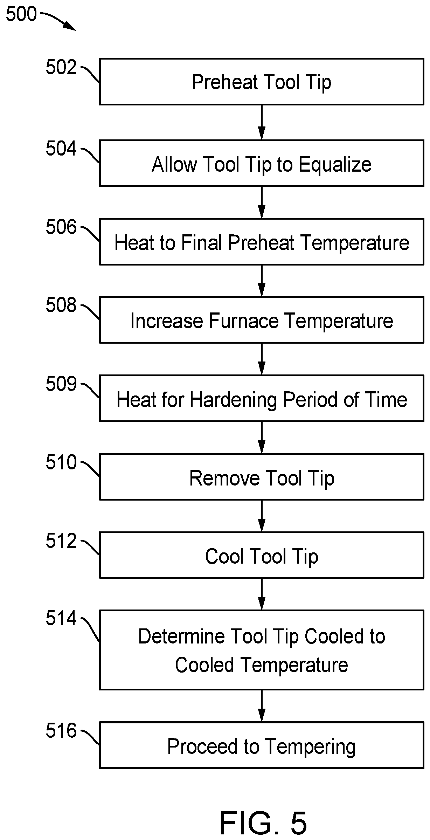

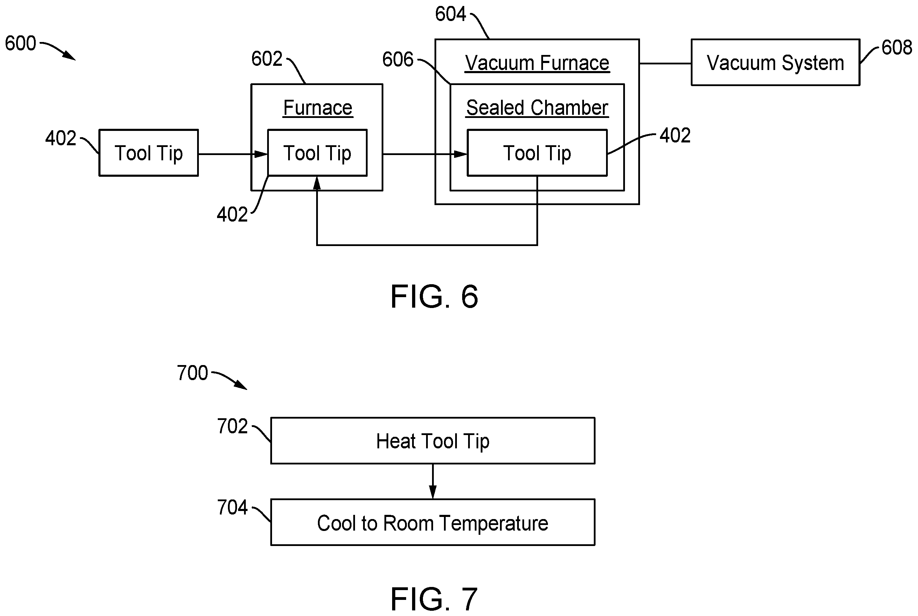

Figure 5 depicts a flowchart 500 for hardening a replaceable tool tip 402 according to the disclosed embodiments. Flowchart 500 may refer to Figures 1-4 and 6 for illustrative purposes. Flowchart 500, however, is not limited to the embodiments disclosed by Figures 1-4 and 6. Figure 6 depicts a block diagram of a system 600 for hardening replaceable tool tip 402 according to the disclosed embodiments.

Flowchart 500 discloses a process associated with tool tip 402 being comprised of tool steel. In some embodiments, the tool steel may be D2 steel. Other embodiments may implement H13 or P20 steel. Thus, the parameters disclosed below, such as temperature or a period of time, may differ depending on the type of tool steel used for tool tip 402. The parameters are limited to the values disclosed below. Further, tool tip 402 should achieve a hardness of Rockwell Hardness Scale C (HRC) 58-62, which provides a desired balance of hardness or wear resistance and toughness.

Step 502 executes by preheating tool tip 402 within furnace 602. Furnace 602 may heat no faster than about 400 degree Fahrenheit (F), or 204.444 degrees Celsius (C), per hour up to an initial preheat temperature. In some embodiments, the initial preheat temperature may be about 1200 degrees F, or 648.8889 degrees C. Step 504 executes by allowing tool tip 402 to equalize to the initial preheat temperature. Step 506 executes by heating furnace 602 to a final preheat temperature. In some embodiments, the heating operation to the final preheat temperature is performed in one step. The final preheat temperature may be about 1450 degrees F, or 787.7778 degrees C. This step also may continue until the temperature of tool tip 402 equalizes with the final preheat temperature.

Step 508 executes by increasing the temperature in furnace 602 to a hardening temperature. In some embodiments, the hardening temperature may be between 1796 to 2030 degrees F, or 980 to 1100 degrees C. In other embodiments, the hardening temperature may be about 1800 degrees F, or 982.2222 degrees C. Step 509 executes by heating tool tip 402 in furnace 602 for a hardening period of time. Furnace 602 holds tool tip 402 at the hardening temperature until heated through. For example, the whole tip may be heated completely, then the disclosed process holds the temperature for a period of time, such as 15-30 minutes. The hardening period of time held at the hardening temperature may depend on the thickness or dimensions of tool tip 402. This time allows the steel of tool tip 402 to fully absorb the heat to transform the internal structure of tool tip 402. Furnace 602 may heat tool tip 402 to allow the steel to absorb carbon atoms from the atmosphere.

Step 510 executes by removing tool tip 402 from furnace 602 and being placed in vacuum furnace 604. Vacuum furnace 604 heats materials within a sealed chamber 606 that has been evacuated to create a vacuum. The controlled environment may prevent oxidation and other unwanted reactions to allow for precise heat treatment processes. A vacuum system 608 may pump out the air and other gases from sealed chamber 606.

Step 512 executes by cooling tool tip 402 within vacuum furnace 604. Tool tip 402 may cool down in this step to a cooled temperature. Step 514 executes by determining tool tip 402 is cooled to the cooled temperature. In some embodiments, the cooled temperature is about 150 degrees F, or 65.5556 degrees C. Once tool tip 402 cools to the cooled temperature, it is removed from vacuum furnace 604. Step 516 executes by proceeding to the tempering process disclosed in greater detail below.

Figure 7 depicts a flowchart 700 for tempering replaceable tool tip 402 according to the disclosed embodiments. Flowchart 700 may refer to Figures 1-6 for illustrative purposes. Flowchart 700, however, is not limited to the embodiments disclosed by Figures 1-6. According to the disclosed embodiments, prompt tempering may reduce internal stresses. The process of tempering improves hardness and elasticity of the steel of tool tip 402.

Flowchart 700 disclosed a process associated with tool tip 402 being comprised of tool steel. In some embodiments, the tool steel may be D2 steel. Other embodiments may implement H13 or P20 steel. Thus, the parameters disclosed below, such as temperature or a period of time, may differ depending on the type of tool steel used for tool tip 402. The parameters are limited to the values disclosed below. Further, tool tip 402 should achieve a hardness of Rockwell Hardness Scale C (HRC) 58-62, which provides a desired balance of hardness or wear resistance and toughness.

Step 702 executes by heating tool tip 702 in furnace 602 to a tempering temperature for a tempering period of time. In some embodiments, the tempering temperature is about 525 degrees F, or 273.889 degrees C. The tempering period of time may be about two hours. An additional hour may be added to the tempering period of time for every inch of thickness of tool tip 402 over two inches. This step may balance the hardness and toughness of tool tip 402, for Rockwell C 58-60.

Step 704 executes by cooling tool tip 402 in furnace 602 to a room temperature. In some embodiments, the room temperature may vary according to the room, but may be about 60 to 75 degrees F, or 15.5556 to 23.8889 degrees C. Using the hardening and tempering processes disclosed by flowcharts 500 and 700, tool tip 402, using D2 steel, is treated to get a targeted hardness and toughness.

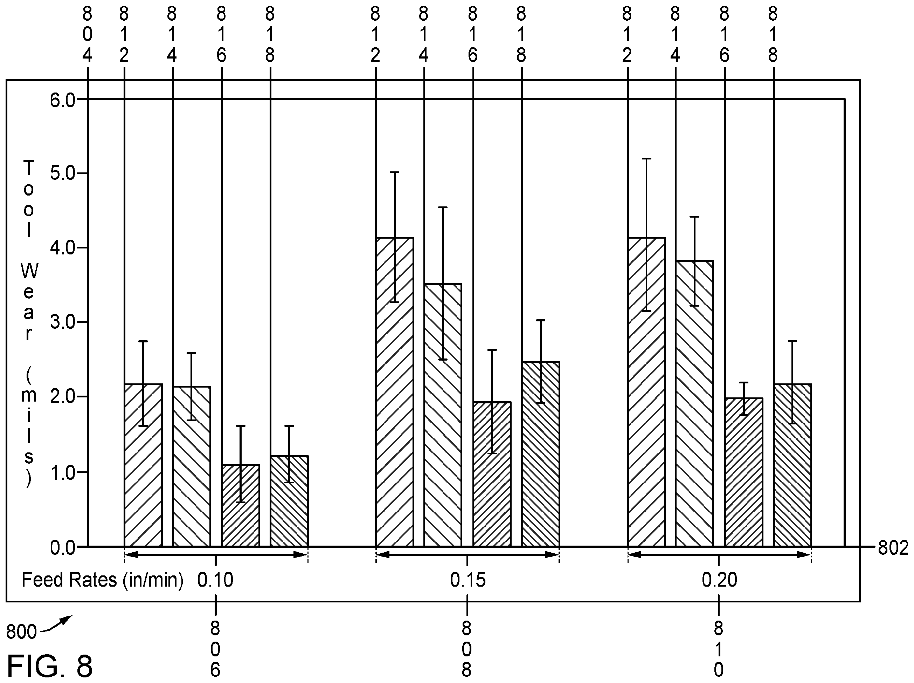

Figure 8 depicts a graph 800 of the tool wear comparison between annealed/as received tool tips versus heat treated tool tips according to the disclosed embodiments. Cutting trials may be performed to compare the tool tip hardened and tempered as disclosed above to annealed tool tips that are used as is. Axis 802 may represent feed rates in inches/minute for tool 1. Axis 804 may represent tool wear in mils for the tool tips.

Axis 802 may include three different feed rates 806, 808, and 810. Tool tips 402 may include as received tool tip 812, as received tool tip 814, heat treated tool tip 816, and heat treated tool tip 818. Graph 800 includes bars showing the tool wear for tool tips 812-818 at different feed rates 806, 808, and 810. At the same feed rates, tool wear may be reduced up to 50%, which means the heat-treated tool tips having the potential to further increase the material removal rate. When the hardness of the tool tip increases by heat treatment, the penetration of the abrasive grains into the tool may decrease, which results in more abrasive particles in workpiece 23. Thus, tool wear is reduced and material remove rate improved.

For example, referring to feed rate 806, this feed rate may have a value of 0. 10 in/min for tool 1. For as received tool tips 812 and 814, the tool wear is about 2.0 mils. For heat treated tool tips 816 and 818, the tool wear is about 1.0 to 1.25 mils. Feed rate 808 may have a value of 0.15 in/min for tool 1. As received tool tips 812 and 814 have tool wear of about 4.0 mils and 3.5 mils, respectively. Heat treated tool tips 816 and 818 have tool wear of about 2.0 mils and 2.5 mils, respectively. Feed rate 810 may have a value of 0.20 in/min for tool 1. As received tool tips 812 and 814 have tool wear of about 4.0 mils and 3.75 mils, respectively. Heat treated tool tips 816 and 818 have tool wear of about 2.0 mils.

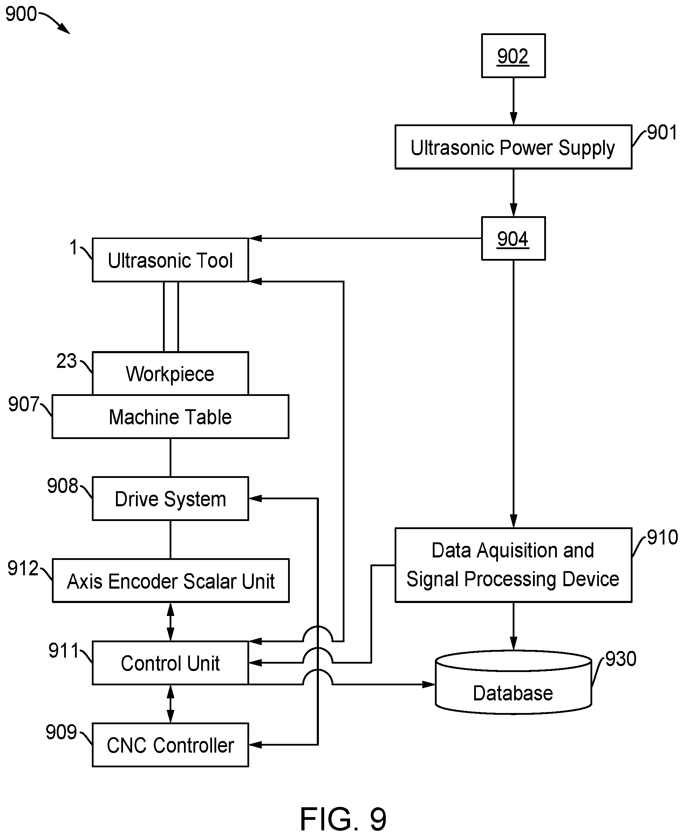

Figure 9 depicts a block diagram of an ultrasonic machining system 900 using ultrasonic vibration tool 1 according to the disclosed embodiments. System 900 includes an ultrasonic power supply 901, which receives incoming electrical power at power terminal 902. Power supply 901 supplies electrical power to ultrasonic vibration tool 1 via power terminal 904. Tool 1 includes tool tip 5 or 402, which is used to perform a machining operation on workpiece 23. Workpiece 23 is mounted on machine table 907, which in turn is driven in x-y-z directions by drive system 908 under the control of CNC controller 909. Ultrasonic power supply 901 may also supply electrical power to drive system 908 and CNC controller 909, or theses elements can be powered from other sources.

When tool 1 is fixed in a stationary position, drive system 908 can position workpiece 23 under tool tip 5 or 402 as needed in order to carry out the machining operation under the control of CNC controller 909. Alternatively, workpiece 23 can remain stationary and tool 1 can be carried by its own drive system similar to drive system 908 also under control of a CNC controller, similar to CNC controller 909.

After appropriate filtering to remove unwanted artifacts, if necessary, a data signal from data acquisition and signal processing device 910 is suppled to control unit 911. Control unit 911 also may control the operation of CNC controller 909. The data signal is used by control unit 911 to control the operation of CNC controller 909 to, in turn, control the feed rates of drive system 908 to maintain desired loads on tool 1. Speed and position data from axis encoder scalar unit 912 also may be supplied in real time to control unit 911. Based on all of this data, control unit 911 can determine the motion and rate of motion along the machining axis of tool 1 required in order to achieve the desired and most efficient machining process. Control unit 911 can also control the operation of tool 1, e.g., turning the tool on and off, controlling various operating parameters, and turning the slurry source on and off.

Database 930 is included in system 900. During the disclosed processes, all process data, including machining conditions, reference and actual load as well as amplitude profiles may be stored in database 930. The data may be saved for analysis. If the targeted process performance is achieved to meet the quality inspection requirements of parts subject to ultrasonic machining by tool 1, then the reference process signature and the measured signature may be fused as an adjusted baseline signature. The adjusted baseline signature may be saved to database 930. In machining subsequent CMC parts, the adjusted baseline signature may be used for process monitoring and control by following the same processes used to machine the previous workpieces.

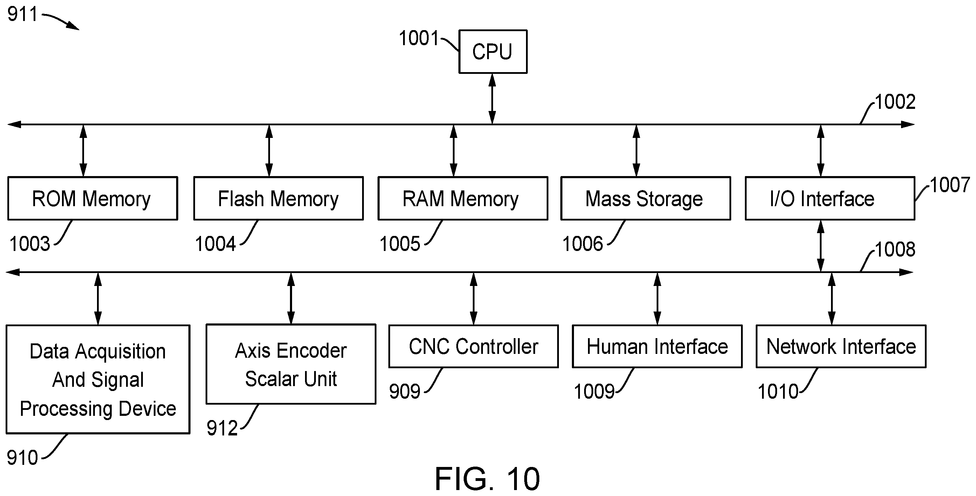

Figure 10 depicts a block diagram of a one embodiment of control unit 911 according to the disclosed embodiments. Control unit 911 includes central processing unit (CPU) 1001, which is used to execute computer software instructions. CPU 1001 is coupled, via bus 1002, to ROM memory 1003, flash memory 1004, RAM memory 1005, mass storage 1006 and I/O interface 1007.

ROM memory 1003 and flash memory 1004 may be used to store computer software instructions for execution by CPU 1001. RAM memory 1005 may also be used for storing computer software instructions, and especially for storing information that is only needed for a short period of time. Mass storage 1006 is used for longer and larger data storage needs as may be required to be retained for data analysis over time.

I/O interface 1007 allows the control unit to communicate via bus 1008 to other parts of the system, such as data acquisition and signal processing device 910, axis encoder scalar unit 912, and CNC controller 909, all shown in Figure 9. Also coupled to control unit 911 may be human interface device 1009, which allows an operator of the system to input control commands to the system via switches, knobs, keyboards and the like and to receive information from the system via lights, displays, audio devices and the like.

A network interface 1010 may also be provided that allows control unit 911 to communicate with Internet hosted services such as Cloud Storage Units, web applications, and the like that consume data provided via control unit 911 for analysis and subsequent retrieval. The functionality disclosed herein also may be integrated into CNC controller 909.

While the present disclosure has been particularly described, in conjunction with specific disclosed embodiments, it is evident that many alternatives, modifications and variations will be apparent to those skilled in the art in light of the foregoing description. It is therefore contemplated that the appended claims will embrace any such alternatives, modifications and variations as falling within the true scope of the present disclosure.

## Claims

1. A method for treating a tool tip of an ultrasonic vibration tool, the method comprising:
preheating the tool tip to an initial preheat temperature within a furnace;
heating the tool tip to a final preheat temperature from the initial preheat temperature within the furnace at once;
increasing heat within the furnace to a hardening temperature for the tool tip for a hardening period of time;
placing the tool tip into a vacuum furnace until the tool tip is cooled to a cooled temperature;
heating the tool tip to a tempering temperature in the furnace for a tempering period; and
allowing the tool tip to cool in the furnace to a room temperature to achieve a hardness of Rockwell Hardness Scale C (HRC) value between 58 and 62.

2. The method of claim 1, wherein the tool tip is comprised of tool steel, wherein, optionally, the tool tip is comprised of one of H13, P20, and D2 steel.

3. The method of claim 1 or 2, wherein the initial preheat temperature is between 640 degrees and 650 degrees Celsius.

4. The method of claim 1, 2 or 3, wherein preheating the tool tip includes increasing a temperature within the furnace to the initial preheat at a specified increment per hour.

5. The method of any preceding claim, wherein the final preheat temperature is between 780 degrees and 790 degrees Celsius.

6. The method of any preceding claim, wherein the hardening temperature is between 980 degrees and 990 degrees Celsius.

7. The method of any preceding claim, wherein the cooled temperature is between 60 degrees and 70 degrees Celsius.

8. The method of any preceding claim, wherein the tempering temperature is between 270 degrees and 280 degrees Celsius.

9. The method of any preceding claim, wherein the tempering period corresponds to a thickness of the tool tip.

10. The method of any preceding claim, wherein the vacuum furnace includes a sealed chamber to hold the tool tip.

11. An ultrasonic vibration tool comprising:
a transducer configured to convert electrical energy into mechanical vibrations;
a horn coupled to the transducer to amplify the mechanical vibrations; and
an ultrasonic tool coupled to the horn to move along a longitudinal axis of the ultrasonic vibration tool according to the mechanical vibrations, wherein the ultrasonic tool includes a tool tip comprised of heat-treated steel.

12. The ultrasonic vibration tool of claim 11, wherein the tool tip is replaceable.

13. The ultrasonic vibration tool of claim 11 or 12, wherein the steel comprises tool steel.

14. The ultrasonic vibration tool of claim 11, 12 or 13, wherein the horn is comprised of a titanium alloy.

15. An ultrasonic machining system comprising:
the ultrasonic vibration tool of any of claims 11 to 14;
a slurry component having a slurry muzzle to deliver a slurry having abrasive particles in an area of the tool tip, wherein the tool tip engages the abrasive particles to machine a workpiece; and
an ultrasonic power supply to provide the electrical energy to the ultrasonic vibration tool,
wherein, optionally, the ultrasonic machining system further comprises a control unit to control the ultrasonic vibration tool.
